# EUROPEAN PATENT APPLICATION

(11) **EP 4 691 388 A1**
(43) Date of publication of application: **11.02.2026**
(21) Application number: 24193561.8
(22) Date of filing: 08.08.2024
(51) Int. Cl.: A61B 17/34, A61B 5/00, A61B 5/02, A61B 5/29, A61N 1/372, A61N 1/375

(54) **IMPLANTATION TOOL, AND METHOD FOR PREPARING AN IMPLANTATION TOOL**

(71) Applicant: BIOTRONIK SE & Co. KG, 12359 Berlin (DE)
(72) Inventor: WHITTINGTON, R. Hollis, Portland, 97202 (US); KRAETSCHMER, Hannes, West Linn, 97068 (US); GRAUHAN, Ole, 14163 Berlin (DE)
(74) Representative: Biotronik Corporate Services SE

(57) **Abstract**

An implantation tool (20) for implanting an implantable medical device (22) is provided. The implantation tool (20) comprises: a first axial section (32) for being grabbed by an implanter, with an outer surface of the first axial section (32) having a first friction resistance; and a second axial section (34) for being introduced in a body tissue of a patient, wherein an outer surface of the second axial section (34) has a second friction resistance which is less than the first friction resistance.

## Description

The present invention refers to an implantation tool and to a method for preparing an implantation tool. In particular, the present invention refers to an implantation tool for implanting an implantable medical device, and to a method for preparing the implantation tool, e.g. into a body tissue of a body, e.g. a human body.

Implantable Medical Devices (IMDs) may be implantable pacemakers or Implantable Cardiac Monitors (ICMs). ICMs are tools which may be used for diagnosis of unknown arrhythmias, palpitations, identification of Atrial Fibrillation (AF), and other arrhythmias of a human or animal body. ICMs currently are becoming more commonplace and an insertion of the ICMs is more frequently being performed outside of an operating room, in particular in procedure rooms and even doctor's offices. Therefore, it is important to simplify the implantation procedure, during which the IMD is inserted into the corresponding body tissue, as much as possible.

An implantation tool facilitates the implantation procedure such that it easily may be carried out by a physician or even a medical assistant, in other words by an implanter. During the implantation procedure, the implantation tool may have to cut and thereby open the body tissue, in particular the skin, and to spread the body tissue such that a tunnel is formed under the skin within the body tissue for accommodating the IMD and at least a part of the implantation tool itself.

During the implantation procedure, the implanter has to exert a force on the implantation tool which is transferred to the body of the patient. The patient may feel a corresponding tugging and pushing sensations at the skin and/or body tissue, which may be uncomfortable. This creates a negative experience for the implanter and for the patient. In some cases, an unintentional tissue damage may be caused because of the exerted force, increasing the negative experience for the implanter and for the patient. This may become even worse under conditions of a tight body tissue, body tissue that is not well hydrated, and/or in the presence of anatomical features such as ribs and connective tissue, because the exerted force may have to be increased.

So, any method which may reduce the forces transmitted to the patient during the implantation procedure will ease the procedure and make it more likely to be accepted by the implanter and the patient, in particular those with less surgical experience.

A typical implantation tool may be composed of a first axial section comprising a locking handle unit for being grabbed and handled by the implanter, and a second axial section comprising a clamshell with top and bottom halves. The clamshell portion may be configured for forming an incision into the skin of the patient and correspondingly separating the skin. Then, the clamshell portion may be configured for being inserted into the body tissue at the incision, and for performing a function of spreading the body tissue. The clamshells may comprise or may be made of plastic. The clamshells may be configured for withstand and transfer the force exerted during the implantation procedure. Further, one or more sharp edges may be provided at a tip of the clamshell, wherein the sharp edges may be provided for cutting through and opening the body tissue such that not so much force has to be exerted for implanting the IMD.

It is an object of the present invention to provide an implantation tool for implanting an implantable medical device, which contributes to a positive experience for a corresponding implanter and/or patient or which at least weakens the above-mentioned negative experience, which contributes to avoid or at least to reduce an unintentional tissue damage, and/or which may enable a fast, easy, and/or comfortable implantation procedure.

It is another object of the present invention to provide a method for implanting an implantable medical device, which contributes to a positive experience for a corresponding implanter and/or patient or which at least weakens the above-mentioned negative experience, which contributes to avoid or at least to reduce an unintentional tissue damage, and/or which may be carried out fast, and/or in an easy and/or comfortable way.

Such objects may be met with the subject-matter of the independent claims. Advantageous embodiments are defined in the dependent claims as well as the corresponding specification and figures.

According to a first aspect, the object is achieved by an implantation tool for implanting an implantable medical device. The implantation tool has a first axial section for being grabbed by an implanter, with an outer surface of the first axial section having a first friction resistance; and a second axial section for being introduced in a body tissue of a patient, wherein an outer surface of the second axial section has a second friction resistance which is less than the first friction resistance.

The reduced and low second friction resistance within the second axial section may reduce frictional forces between the implantation tool and the body tissue during the implantation procedure. This enables to keep a force, which has to be exerted on the implantation tool by an implanter during the implantation procedure, low. This may contribute to a positive experience for a corresponding implanter and/or patient, or at least to a weak negative experience only. The reduced and low second friction resistance within the second axial section may also contribute to avoid or at least to reduce an unintentional tissue damage. In general, the reduced and low second friction resistance within the second axial section may enable a fast, easy, and/or comfortable implantation procedure. These advantages may also refer to that part of the implantation procedure during which the implantation tool is retracted from the body tissue. By reducing the friction between the body tissue and the implantation tool, it may be more acceptable for the implanter to implant a very large and/or long device in order to achieve better properties such as longevity, signal quality, or RF-transmission characteristics since the barrier for the implantation will be lowered. In sum, these advantages may make the implantation procedure easier, may provide more control and sense of safety to the implanter, may enable a better sense of feedback from the body tissue, and/or reduce a risk of breaking the implantation tool due to excessive forces. These advantages may become even more important when the implantation tool is used for implanting an ICM, e.g. an implantable cardiac monitor, because a surface area of the corresponding implantation tool, which comes in contact with the body tissue, may be relatively large, compared to other implantation tools, and because the friction may play a larger role in this case.

The implantation tool is configured for placing the implantable medical device (IMD) in the body tissue at a desired location. The IMD may be an implantable sensor or an Implantable Cardiac Monitor (ICM) (sometimes also referred to as loop recorder). The implantation tool has a portion or region, i.e. the second axial section, that is configured for going into the body tissue and/or under the skin of the body to open a tunnel for the IMD and to place the IMD within the tunnel. The portion and/or region that goes inside the body during the implantation procedure is specifically designed to minimize the friction between the implantation tool and the body tissue upon insertion and removal of the implantation tool. The implantation tool has another portion and/or region, i.e. the first axial section, which may be used to grasp the implantation tool. This other portion and/or region provides the higher first friction resistance for maintaining an ability to securely hold the implantation tool and to control the implantation tool during the implantation procedure.

The above friction resistances refer to the friction resistances between the body tissue and the corresponding axial section of the implantation tool. Of course, the first axial section is not configured for being inserted into the body tissue, nevertheless the first friction resistance may easily be measured with respect to the body tissue. In addition, the first axial section has to be grabbed and handled by the implanter, wherein the implanter may touch the first axial section with the body tissue of his hand, enabling to measure the first friction resistance. In any case, it is advantageous for the implanter when the friction resistance of the first axial section is high, because this facilitates controlling the implantation tool during the implantation procedure. In contrast, the second axial section has to be at least partly inserted into the body tissue of the patient and the low second friction resistance facilitates the implantation procedure, because less force has to be used for implanting the implantable medical device. In addition the low second friction resistance may enhance the experience of the patient, because less pain may be caused by the lower friction between the implantation tool and his or her body tissue. The implantation tool may be formed such that all regions that are to be inserted into the body tissue have the second friction resistance.

The difference in friction resistance between the sections of the implantation tool may be implemented by the use of materials and/or coatings in the sections having a different coefficient of friction.

The implantation tool of the present invention is not configured to be inserted into a blood vessel / not suitable for insertion into a blood vessel. The implantation tool may be configured for subcutaneous insertion into body tissue, in particular into subcutaneous tissue, fatty tissue and/or muscle. The implantation tool may be stiff.

The first friction resistance may be at least 1 N, preferably in a range of 1 N to 10 Ne.g. of 1 N to 8 N, e.g. of 1 N to 6 N. The second friction resistance may be in a range of 0.029 N to 0.245N, e.g. in the range of 0.029 N to 0.2 N, e.g. in the range of 0.029 N to 0.1 N. For example, the second friction resistance may be between 3 % and 50 %, e.g. between 10 % and 25 %, of the first friction resistance. The friction resistances mentioned in this description may be measured in aqueous solution, e.g. distilled water, as pull force to move the respective article through the pad of a lubricity/pinch test applying a pinch force of 4,9 N at room temperature. The first friction resistance may be equivalent to a first friction coefficient of at least 0.1, preferably in a range of 0.13 to 1,5, e.g. in a range of 0.13 to 1,2, e.g. in a range of 0.3 to 1. The second friction resistance may be equivalent to a second friction coefficient in a range of 0.01 to 0.05, e.g. in a range of 0.01 to 0.04, e.g. in a range of 0.01 to 0.03. The friction coefficients mentioned in the this description may be measured by the same lubricity/pinch test as the friction resistances.

The implanter may be a physician and/or medical practitioner. The second axial section may be configured for opening the tunnel in the body tissue, wherein the tunnel may be configured for accompanying the implantable medical device. The implantation tool may extend along a longitudinal axis of the implantation tool. The implantation tool may be at least partly symmetric with respect to the longitudinal axis, e.g. rotationally symmetric. A direction in which the implantable medical device may be implanted into the body tissue may be parallel to the longitudinal axis. The implantation tool may have a clamshell, optionally with top and bottom halves, for separating the body tissue, for spreading the body tissue, and for being inserted into the body tissue.

According to an embodiment, the implantation tool may have a housing providing the first friction resistance within the first axial section and having a third friction resistance at an outer surface of the housing in the second axial section; and a low friction layer arranged on the housing within the second axial section, wherein the low friction layer provides the second friction resistance and forms at least a part of the outer surface of the implantation tool within the second axial section, and wherein the second friction resistance is less than the third friction resistance. The low friction layer may be arranged on an existing implantation tool. Therefore, an existing design of the implantation tool does not have to be changed in order to provide the second axial section having the second friction resistance. Alternatively or additionally, the low friction layer may be easily arranged on the housing in the second axial section, independent thereof whether the design of the implantation tool is new or not.

The first friction resistance may be the same as the third friction resistance. Consequently the first friction coefficient may be the same as the third friction coefficient. Alternatively, the third friction resistance may be less than the first friction resistance. The third friction resistance may be in a range of 0.05 N to 0.8 N, e.g. in the range of 0.05 N to 0.5 N, e.g. in the range of 0.05 N to 0.3 N. Alternatively, the third friction resistance may be equivalent to a third friction coefficient in the range of 0.02 to 0.2, e.g. in the range of 0.02 to 0.1, e.g. in the range of 0.02 to 0.05. The housing may accommodate the implantable medical device to be implanted. The housing may further accommodate one or more mechanical and/or electrical components for implanting the implantable medical device. The housing may have two or more housing elements. For example, the housing may have a first housing element in the first axial section and a second housing element in the second axial section, wherein the housing elements are coupled to each other. The first housing element may be configured for accommodating at least in part the one or more mechanical and/or electrical components for implanting the implantable medical device. The second housing element may be configured for accommodating the implantable medical device to be implanted. The second housing element may have a tip of the implantation tool, wherein the tip may be configured for opening the body tissue and for being introduced into the body tissue first. For example, the tip may have one or more sharp parts, e.g. edges, for cutting the body tissue. Further, the second housing element may be formed such that the body tissue is spread when introducing the second housing element into the body tissue, such that the tunnel is formed. For example, an outer diameter of the second housing element may increase with increasing distance to the tip. The housing may comprise or may be made of plastic. The housing may be formed by molding.

According to an embodiment, the low friction layer is formed by a coating applied on the housing in the second axial section. The coating may be biocompatible such that the body tissue may not be chemically compromised by the coating. The coating may be any coating that is designed to reduce friction and that is biocompatible. The coating may be a hydrophilic coating, a silicon coating, a Polytetrafluoroethylene coating, a lubricant coating, or an oil coating. The coating may be applied on the housing of the implantation tool within the second axial section, for example on an outer surface of the second housing element. The first axial section, e.g. the first housing element, stays free from the coating.

According to an embodiment, the coating is a hydrophilic coating. The hydrophilic coating may be configured such that is dry and not very slippery before being in contact with the body tissue and that it becomes lubricious and/or slippery when being wet because of the contact to the body tissue. Therefore, the friction resistance of the outer surface of the second axial section provided by the hydrophilic coating may be different when the implantation tool is not in contact with the body tissue than when the implantation tool is in contact with the body tissue. For example, the outer surface of the second axial section may have the second friction resistance when being in contact with the body tissue and a fourth friction resistance larger than the second friction resistance when not being in contact with the body tissue, e.g. before the implantation procedure. In particular, the fourth friction resistance may be even larger than the first and/or third friction resistance. In this case, the above mentioned first and second friction resistances may refer to the case where the corresponding axial section is in contact with the, at least somehow wet, body tissue. The hydrophilic coating may be any hydrophilic coating which is commonly used with catheters for reducing a friction force between the catheter and inner walls of the veins, after the catheter is introduced into the veins. It has to mentioned in this context, that it is not yet known to use the hydrophilic coating of the catheters for facilitating conducting the catheter through the body tissue towards the veins.

The hydrophilic coating may be applied to the implantation tool to cover all of the regions that are to be inserted in the body tissue. The coating could be established for instance by spraying or dipping these regions in a corresponding hydrophilic solution. Known hydrophilic and biocompatible coatings may provide an extremely low friction surface, which may be robust enough to withstand multiple implantations, e.g. in case a repositioning of the IMD is necessary. The hydrophilic coating may be acceptable for human use, biocompatible, and consistent with usage with an implantation tool which has temporary contact with the body tissue.

According to an embodiment, the low friction layer is formed by a sheet, film or membrane covering the housing in the second axial section and providing the second friction resistance. The sheet, film, or membrane may be biocompatible such that the body tissue may not be chemically compromised by the sheet, film or, respectively, membrane. The sheet, film, or membrane may be any sheet, film or, respectively, membrane that is designed to reduce friction. The sheet, film, or membrane may comprise silicon or Polytetrafluoroethylene. The sheet, film, or membrane may be arranged on the housing of the implantation tool within the second axial section, for example on an outer surface of the second housing element. The first axial section, e.g. the first housing element, stays free from the sheet, film or, respectively, membrane.

According to an embodiment, the implantation tool may have a housing providing the first friction resistance within the first axial section and having a low friction surface providing the second friction resistance in the second axial section. In other words, the outer surface of the housing in the second axial section, i.e. an outer surface of the second housing element, may be at least partly treated such that it provides the second friction resistance without any additional layer to be applied and/or arranged on the second housing element.

According to an embodiment, the low friction surface is etched and/or polished. For example, in the case of an extremely low friction surface, there is only a one-time cost of a polished mold to produce a very glossy low friction surface. Alternatively, batch surface treatments may be used to achieve a smooth surface. Whether the low friction surface is etched or polished refers to a method for manufacturing the implantation tool, in particular to a method step in which the low friction surface is provided. However, the etched and/or, respectively, polished low friction surface may be easily differentiated from a surface which is not etched and/or, respectively, polished, e.g. by known microscopic methods. Therefore, whether the low friction surface is an etched low friction surface or a polished low friction surface may also be seen as features of the implantation tool itself.

According to a second aspect, the object is achieved by a method for preparing the implantation tool for implanting the implantable medical device, the method comprising: providing the implantation tool having a first axial section for being grabbed by the implanter, with the outer surface of the first axial section having the first friction resistance, and the second axial section for being introduced in the body tissue of the patient; and treating the second axial section such that the outer surface of the second axial section has the second friction resistance which is less than the first friction resistance.

The features, advantages and embodiments of the implantation tool explained above may easily be transferred to features, advantages and embodiments of the method for preparing the implantation tool.

According to an embodiment, the treating of the second axial section may involve arranging the low friction layer on the housing of the implantation tool within the second axial section, wherein the low friction layer provides the second friction resistance and forms at least in part the outer surface of the second axial section, wherein the second friction resistance is less than the third friction resistance of the housing in the second axial section.

According to an embodiment, the arranging of the low friction layer may involve applying the coating on the housing in the second axial section, wherein the coating forms the low friction layer. The coating may be applied by spray coating or by dipping the second axial section into a solution comprising the coating.

According to an embodiment, the coating is the hydrophilic coating. Hydrophilic dip coating may be an inexpensive addition to product manufacture that would greatly enhance the user experience and encourage use of the implantation tool.

According to an embodiment, the arranging of the low friction layer may involve covering the housing in the second axial section with the sheet, film or membrane forming the low friction layer.

According to an embodiment, the treating of the second axial section may involve treating the outer surface of the housing of the implantation tool in the second axial section such that the low friction surface is formed in the second axial section of the housing, wherein the low friction surface provides the second friction resistance.

The roughness of the low friction surface may be less than a roughness of the housing outside of the low friction surface.

According to an embodiment,the low friction surface is formed by etching and/or polishing the housing within the second axial section. Alternatively, batch surface treatments may be used to form the low friction surface.

In the following, advantageous embodiments of the invention will be described with reference to the enclosed drawings. However, neither the drawings nor the description shall be interpreted as limiting the invention.
- Fig. 1: shows exemplary embodiments of an implantable medical device and of an implantation tool for implanting the implantable medical device.
- Fig. 2: shows a flow chart of an exemplary embodiment of a method for preparing the implantation tool.

The figures are only schematic and not to scale. Same reference signs refer to same or similar features.

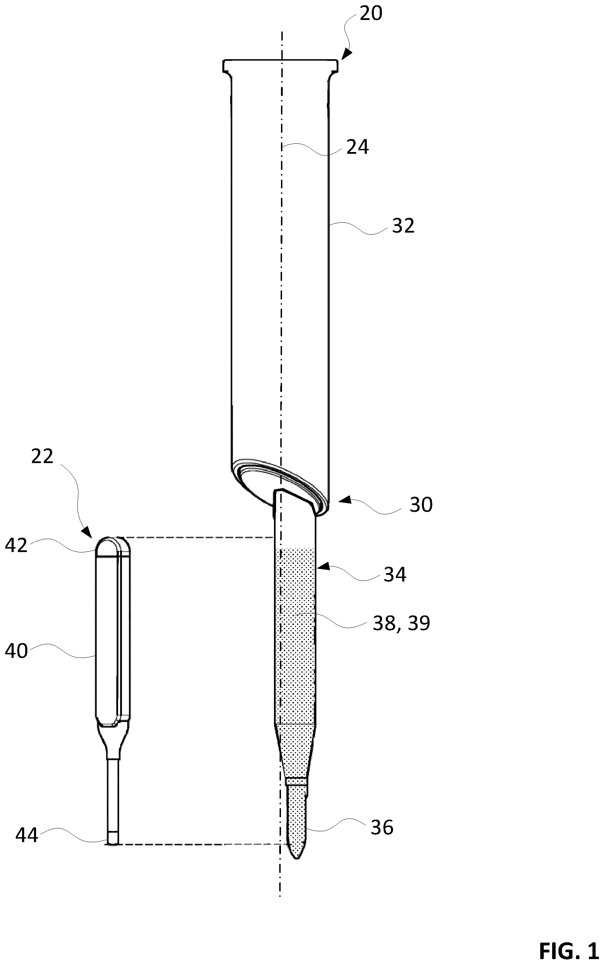

Fig. 1 shows exemplary embodiments of an implantable medical device 22 and of an implantation tool 20 for implanting the implantable medical device 22. The implantation tool 20 may be configured for placing the implantable medical device (IMD) 22 in the body tissue at a desired location. The IMD 22 may be an impantable sensor or an Implantable Cardiac Monitor (ICM).

The implantation tool 20 has a first axial section 32 for being grabbed by an implanter (not shown). An outer surface of the first axial section 32 has a first friction resistance. The implantation tool 20 has a second axial section 34 for being introduced in a body tissue of a patient (not shown). An outer surface of the second axial section 34 has a second friction resistance which is less than the first friction resistance. The implantation tool 20 has a portion or region, i.e. the second axial section 34, that is configured for going into the body tissue and/or under the skin of the body to open a tunnel for the IMD 22 and to place the IMD 22 within the tunnel. The portion and/or region that goes inside the body during a corresponding implantation procedure is specifically designed to minimize the friction between the implantation tool 20 and the body tissue upon insertion and removal of the implantation tool 20. The implantation tool 20 has another portion and/or region, i.e. the first axial section 32, which may be used to grasp the implantation tool 20. This other portion and/or region provides the higher first friction resistance for maintaining an ability to securely hold the implantation tool 20 and to control the implantation tool 20 during the implantation procedure.

The implantation tool 20 may extend along a longitudinal axis 24 of the implantation tool 20. The implantation tool 20 may be at least partly symmetric with respect to the longitudinal axis 24, e.g. rotationally symmetric. For example, the first axial section 32 may be at least partly rotationally symmetric with respect to the longitudinal axis 24. A direction in which the implantable medical device 22 may be implanted into the body tissue may be parallel to the longitudinal axis 24.

The implantation tool 20 may have a housing 30 providing the first friction resistance within the first axial section 32 and having a third friction resistance at an outer surface of the housing 30 in the second axial section 34. The housing 30 may accommodate the implantable medical device 22 to be implanted. The housing 30 may further accommodate one or more mechanical and/or electrical components for implanting the implantable medical device 22. The housing 30 may have two or more housing elements. For example, the housing may have a first housing element in the first axial section 32 and a second housing element in the second axial section 34, wherein the housing elements are coupled to each other. The first housing element may be configured for accommodating at least in part the one or more mechanical and/or electrical components for implanting the implantable medical device 22. The second housing element may be configured for accommodating the implantable medical device 22 to be implanted. In case of the implantation tool 20 having a clamshell portion (not shown) configured for forming an incision into the skin of the patient and correspondingly separating the skin, the clamshell portion may be provided by the second housing element.

The second housing element may have a tip 36 of the implantation tool 20. The tip 36 may be configured for opening the body tissue and for being introduced into the body tissue first. For example, the tip 36 may have one or more sharp parts, e.g. edges, for cutting the body tissue. Further, the second housing element may be formed such that the body tissue is spread when introducing the second housing element into the body tissue, such that the tunnel is formed. For example, an outer diameter of the second housing element increases with increasing distance to the tip 36. The housing 30 may comprise or may be made of plastic. The housing 30 may be formed by molding.

A low friction layer 38 may be arranged on the housing 30 within the second axial section 34, wherein the low friction layer 38 may provide the second friction resistance and may form at least a part of the outer surface of the implantation tool 20 within the second axial section 34. The second friction resistance may be less than the third friction resistance. So, the low friction layer 38 lowers the friction resistance of the outer surface of the implantation tool 20 within the second axial section 34. The first friction resistance may be the same as the third friction resistance. Alternatively, the third friction resistance may be less than the first friction resistance.

The low friction layer may be formed by a coating applied on the housing 30 in the second axial section 34. The coating may be biocompatible such that the body tissue may not be chemically compromised by the coating. The coating may be any coating that is designed to reduce friction and that is biocompatible. The coating may be a hydrophilic coating, a silicon coating, a Polytetrafluoroethylene coating, a lubricant coating, or an oil coating. The coating may be applied on the housing 30 of the implantation tool 20 within the second axial section 34, for example on an outer surface of the second housing element. The first axial section 32, e.g. the first housing element, stays free from the coating.

In case of the hydrophilic coating, the hydrophilic coating may be configured such that is dry and not very slippery before being in contact with the body tissue and that it becomes lubricious and/or slippery when being wet because of the contact to the body tissue. Therefore, the second friction resistance of the outer surface of the second axial section 34 provided by the hydrophilic coating may be different when the implantation tool 20 is not in contact with the body tissue than when the implantation tool 20 is in contact with the body tissue. For example, the outer surface of the second axial section 34 may have the second friction resistance when being in contact with the body tissue and a fourth friction resistance larger than the second friction resistance when not being in contact with the body tissue, e.g. before the implantation procedure. In particular, the fourth friction resistance may be even larger than the first and/or third friction resistance. In this case, the above mentioned first and second friction resistances may refer to the case where the corresponding axial section 32, 34 is in contact with the, at least somehow wet, body tissue.

The first friction resistance may be in a range of 1 N to 10 Ne.g. of 1 Nto 8 N, e.g. of 1 N to 6 N. The second friction resistance may be in a range of 0.029 N to 0.245N, e.g. in the range of 0.029 N to 0.2 N, e.g. in the range of 0.029 N to 0.1 N. The third friction resistance may be in a range of 0.05 N to 0.8 N, e.g. in the range of 0.05 N to 0.5 N, e.g. in the range of 0.05 N to 0.3 N. For example, the second friction resistance may be between 3 % and 50 %, e.g. between 10 % and 25 %, of the first friction resistance.

The hydrophilic coating may cover all of the regions of the implantation tool 20 that are to be inserted in the body tissue. The coating could be established for instance by spraying or dipping these regions in a corresponding hydrophilic solution. The hydrophilic and biocompatible coating may provide an extremely low friction surface, which may be robust enough to withstand multiple implantations, e.g. in case a repositioning of the IMD 22 is necessary. The hydrophilic coating may be acceptable for human use, biocompatible, and consistent with usage with an implantation tool 20 which has temporary contact with the body tissue.

As an alternative to the coating, the low friction layer 38 may be formed by a sheet, film or membrane covering the housing 30 in the second axial section 32 and providing the second friction resistance. The sheet, film, or membrane may be biocompatible such that the body tissue may not be chemically compromised by the sheet, film or, respectively, membrane. The sheet, film, or membrane may be any sheet, film or, respectively, membrane that is designed to reduce friction. The sheet, film, or membrane may comprise silicon or Polytetrafluoroethylene. The sheet, film, or membrane may be arranged on the housing 30 of the implantation tool 20 within the second axial section 34, for example on an outer surface of the second housing element. The first axial section 32, e.g. the first housing element, stays free from the sheet, film or, respectively, membrane.

As an alternative to the low friction layer 38, the housing 30 may have a low friction surface 39 providing the second friction resistance in the second axial section 34. In other words, the outer surface of the housing 30 in the second axial section 34, i.e. an outer surface of the second housing element, may be at least partly treated such that it provides the second friction resistance without any additional layer to be applied and/or arranged on the second housing element. The roughness of the low friction surface 39 may be less than a roughness of the housing 30 outside of the low friction surface. The roughness may be measured in accordance with ISO 4287:1997.

The low friction surface 39 may be provided by etching and/or polishing the outer surface of the housing 30 within the second axial section 34. Alternatively, batch surface treatments may be used to achieve the low friction surface 39.

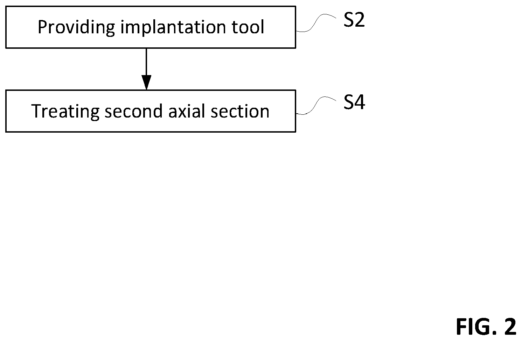

Fig. 2 shows a flow chart of an exemplary embodiment of a method for preparing the implantation tool 20. The method may be carried out at one of the last steps, e.g. at the last step, of a known method for manufacturing the implantation tool 20. For example, the method may be used for finalizing the manufacturing of the implantation tool 20 such that it is ready to use. Alternatively, the method may be carried at an existing implantation tool 20, e.g. an implantation tool 20 which is ready to use, in order to facilitate the implantation procedure and to enhance the experience of the patient, as explained above.

In a step S2, the implantation tool 20 may be provided, for example by manufacturing the implantation tool 20 or by using an existing implantation tool 20. The implantation tool 20 has the first axial section 32 for being grabbed by the implanter, with the outer surface of the first axial section 32 having the first friction resistance, and the second axial section 34 for being introduced in the body tissue of the patient.

In a step S2, the second axial section 34 may be treated such that the outer surface of the second axial section 34 has the second friction resistance which is less than the first friction resistance.

The treating of the second axial section 34 in step S2 may involve arranging the low friction layer 38 on the housing 30 of the implantation tool 20 within the second axial section 34, wherein the low friction layer 38 provides the second friction resistance and forms at least in part the outer surface of the second axial section 34. The arranging of the low friction layer 38 may comprise applying the coating on the housing 30 in the second axial section 34, wherein the coating forms the low friction layer 38. The coating may be applied by spray coating or by dipping the second axial section 34 into a solution comprising the coating. The coating may be the hydrophilic coating. Alternatively, the arranging of the low friction layer 38 may comprise covering the housing 30 in the second axial section 34 with the sheet, film or membrane forming the low friction layer 38.

Alternatively, the treating of the second axial section 34 may comprise treating the outer surface of the housing 30 of the implantation tool 20 in the second axial section 34 such that the low friction surface 38 is formed in the second axial section 34 of the housing 30, wherein the low friction surface 39 provides the second friction resistance. The low friction surface 39 may be formed by etching and/or polishing the housing 30 within the second axial section 34. Alternatively, batch surface treatments may be used to form the low friction surface 39.

Finally, it should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

### List of Reference Numerals

- 20: implantation tool
- 22: implantable medical device
- 24: longitudinal axis
- 30: housing
- 32: first axial section
- 34: second axial section
- 36: tip
- 38: low friction layer
- 39: low friction surface
- 40: main body
- 42: first electrode
- 44: second electrode
- S2: first step
- S4: second step

## Claims

1. An implantation tool (20) for implanting an implantable medical device (22), comprising:
a first axial section (32) for being grabbed by an implanter, with an outer surface of the first axial section (32) having a first friction resistance; and
a second axial section (34) for being introduced in a body tissue of a patient, wherein an outer surface of the second axial section (34) has a second friction resistance which is less than the first friction resistance.

2. The implantation tool (20) of claim 1, comprising:
a housing (30) providing the first friction resistance within the first axial section (32) and having a third friction resistance at an outer surface of the housing (30) in the second axial section (34); and
a low friction layer (38) arranged on the housing (30) within the second axial section (34), wherein the low friction layer (38) provides the second friction resistance and forms at least a part of the outer surface of the implantation tool (20) within the second axial section (34), and wherein the second friction resistance is less than the third friction resistance.

3. The implantation tool (20) of claim 2, wherein the low friction layer (38) is formed by a coating applied on the housing (30) in the second axial section (34).

4. The implantation tool (20) of claim 3, wherein the coating is a hydrophilic coating.

5. The implantation tool (20) of claim 2, wherein the low friction layer (38) is formed by a sheet, film or membrane covering the housing (30) in the second axial section (34) and providing the second friction resistance.

6. The implantation tool (20) of claim 1, comprising a housing (30) providing the first friction resistance within the first axial section (32) and having a low friction surface (39) providing the second friction resistance in the second axial section (34).

7. The implantation tool (20) of any one of claims 6, wherein the low friction surface (39) is etched and/or polished.

8. A method for preparing an implantation tool (20) for implanting an implantable medical device (22), the method comprising:
providing the implantation tool (20) having a first axial section (32) for being grabbed by an implanter, with an outer surface of the first axial section (32) having a first friction resistance, and a second axial section (34) for being introduced in a body tissue of a patient; and
treating the second axial section (34) such that an outer surface of the second axial section (34) has a second friction resistance which is less than the first friction resistance.

9. The method of claim 8, wherein the treating of the second axial section (34) comprises:
arranging a low friction layer (38) on a housing (30) of the implantation tool (20) within the second axial section (34), wherein the low friction layer (38) provides the second friction resistance and forms at least in part the outer surface of the second axial section (34), wherein the second friction resistance is less than a third friction resistance of the housing (30) in the second axial section (34).

10. The method of claim 9, wherein the arranging of the low friction layer (38) comprises:
applying a coating on the housing (30) in the second axial section (34), wherein the coating forms the low friction layer (38).

11. The method of claim 10, wherein the coating is a hydrophilic coating.

12. The method of claim 9, wherein the arranging of the low friction layer (38) comprises covering the housing (30) in the second axial section (34) with a sheet, film or membrane forming the low friction layer (38).

13. The method of claim 8, wherein the treating of the second axial section (34) comprises treating an outer surface of a housing (30) of the implantation tool (20) in the second axial section (34) such that a low friction surface (39) is formed in the second axial section (34) of the housing (30), wherein the low friction surface (39) provides the second friction resistance.

14. The method of 13, wherein the low friction surface (39) is formed by etching and/or polishing the housing (30) within the second axial section (34).
